# EUROPEAN PATENT SPECIFICATION

(11) **EP 1 541 634 B1**
(45) Date of publication and mention of the grant of the patent: **22.10.2008**
(21) Application number: 05101795.2
(22) Date of filing: 07.02.2001
(51) Int. Cl.: C08L 89/06, C08J 3/12, A61K 38/17

(54) **Process for the preparation of micronised collagen, and its therapeutic applications**
Verfahren zur Herstellung von mikronisiertem Kollagen, und therapeutische Verwendungen
Procédé de préparation de collagène micronisé, et ses applications thérapeutiques

(30) Priority: 15.02.2000 IT MI20000246
(43) Date of publication of application: 15.06.2005
(62) Divisional of application: 01915198.4
(73) Proprietor: EURORESEARCH S.r.L., I-20145 Milano (IT)
(72) Inventor: Furlan Diego, 20090 Segrate (IT); Cappelletti Leonardo, 20052 Monza (IT)
(74) Representative: Pistolesi, Roberto

(56) References cited:
- DATABASE CHEMABS [Online] CHEMICAL ABSTRACTS SERVICE, COLOMBUS, OHIO, US; GOTO KOICHI ET AL.: "Powdered collagen and its manufacture" XP002167734 retrieved from STN Database accession no. 124:292849 & JP 08 027035 A (HOKUYOO KK) 30 January 1996 (1996-01-30)

## Description

The present invention relates to the use of micronised collagen having a particle size of not more than 20 microns for the manufacture of a pharmaceutical composition.

Collagen, a polypeptide substance having a molecular weight of approximately 130,000 daltons, is the most abundant fibrous protein in the higher vertebrates because it is the principal constituent of the skin, the connective tissue and the organic material present in the bones and teeth, and represents approximately one third of the total amount of proteins in the human body (Merck Index, Version 12:1, 2543, 1996).

The production of collagen in the bodies of mammals is preceded by the formation of a larger biosynthetic precursor, precollagen, which is then degraded by specific enzymes to form collagen.

Various types of collagen occur naturally and they are all composed of three polypeptide chains which have a constant periodicity and which are arranged in a triple helix; the difference between the various types of collagen is caused by small differences in the primary structure of the chains.

Type I collagen, which is the basic constituent of the skin, bones and tendons, may be regarded as the most abundant of the various types of collagen; it has a 2α1 (I) α2 (I) chain composition where the two α1 chains and the α2 chain are homologous. Present between the two α1 chains and the α2 chain are electrostatic interactions and hydrogen bonds which, together with the presence of hydroxyproline, confer on the molecule characteristics of toughness and strength.

The literature discloses the use of collagen as a stimulating agent in the process of wound-healing by interaction with various growth factors, for its action of capturing fibronectin, as well as the migration and replication of cells which are the consequence thereof (Il collagene nella cicatrizzazione (Collagen in wound-healing) by B. Palmieri, published by Artestampa, January 1990, pages 40-42), and for other actions which have not yet been sufficiently clarified.

In therapeutics, collagen is currently used as a wound-healing agent in clinical surgery, in the treatment of burns, as a vehicle, in surgical prosthesis (suture threads, gauzes, etc.), as a material for implantation, or as a raw material of creams and ointments in the pharmaceutical and cosmetics sector (Beghè, Mian and Palmieri in *Collageno e cicatrizzazione* gauzes, etc.), as a material for implantation, or as a raw material of creams and ointments in the pharmaceutical and cosmetics sector (Beghè, Mian and Palmieri in Collageno e cicatrizzazione "Realtà e prospettive terapeutiche"(Collagen and wound-healing "Facts and therapeutic perspectives"), Istanbul, 1990; Mian & Mian, Topical collagen and wound Healing, 1992, supplement to vol. XIV; Int.J of tissue reactions, Palmieri, Trabucchi and Zucchelli, Collagene e cicatrizzazione, (Collagen and wound-healing) 1993, Tacchi Ed. Pisa; GELFIX^{®} technical gazette of Euroresearch Srl).

Precisely for its characteristics of toughness and strength, the collagen normally used in those sectors is type I collagen.

Collagen is normally obtained in the stable and non-denatured form currently on the market by extraction and purification processes from animal organs, such as, for example, described in JP 2886164. The collagen so obtained is normally a gel which contains from 0.1 to 2.0% of collagen and which, in order then to be used in the various therapeutic applications indicated above, is normally subjected to further conversions; it is, for example, converted by lyophilisation into a platelet having a water content of approximately 17%, or, by drying, into a lamellar structure having a water content of approximately 20%.

The powdered collagen currently on the market has, however, disadvantages and defects of not inconsiderable importance because it is available only in a coarse particle size (>500 microns) which does not enable it to adhere to moist surfaces and prevents it from being used in the form of a spray.

However, it will be appreciated that it is important to be able to rely on a collagen which, as far as possible, is non-denatured, anallergic, free from undesired impurities or contaminants and, above all, is in a finely micronised form, that is to say, in order to enable it to be applied to moist and irregular surfaces, such as, for example, the epidermis or damaged tissue.

The object of the present invention is therefore to obtain a product which, while maintaining the typical characteristics of collagen as regards its wound-healing activity, permits easy, simple and rapid application, is easy and practical to use, can be applied to areas of the body which are difficult of access (for example cavities and recesses), and is sterile and structurally homogeneous.

The above has now been obtained by a particular micronisation process which constitutes one of the subjects of the present invention and which enables powdered collagen having a particle size of not more than 20 microns to be obtained.

The micronisation process according to the present invention utilises the normal atomisers currently on the market, such as the rotary cyclone atomisers produced by Niro A/S and described, for example, in United States patents US 5,632,100, US 5,615,493, US 4,490,403, US 4,369,091 and US 3,956,521, which are incorporated herein by reference.

The operation of those atomisers is well known to persons skilled in the art and therefore will not be discussed in detail here; it is, however, important to underline that they basically comprise a cyclone structure where a solution of the product to be micronised is introduced through a rotating nozzle which brings about the nebulisation thereof and is struck by an ascending stream of an inert gas, generally air, heated to a temperature of the order of from 150 to 400°C.

In the present process, however, a 0.1 - 0.8% by weight/volume aqueous solution of collagen having a pH of from 3.0 to 6.0 is introduced into a normal atomiser and struck by a stream of gas having a temperature substantially lower than those used in the usual micronisation processes; the stream of inert gas, generally air, in fact has a temperature lower than 120°C, preferably of from 70 to 120°C, and even more preferably from 80 to 100°C.

The aqueous collagen solution, generally obtained by diluting a 1.0 - 2.0% by weight/volume gel of type I native collagen with slightly acidic water, preferably has a final pH of from 4 to 5 and a content of collagen of from 0.3 to 0.5% by weight/volume; the collagen powder is then preferably collected in a closed container which is in a form such that the powder maintains a moisture content of less than 15%.

The product so obtained is a collagen powder having a particle size of from 5 to 30 microns, generally not more than 20 microns and preferably of approximately 18 microns, which maintains intact the quaternary aggregation form (in bundles of fibrils) typical of native collagen; the powder so obtained can then be divided up, sterilised and placed in suitable containers (spray dispensers, sachets, bottles, etc.) according to methods known in the art.

The particle size of not more than 20 microns permits both optimum adhesion of the collagen to the wound surface and its use in metering systems in spray dispensers. This last aspect, in particular, is a very important characteristic of the present invention because the spray formulation permits the production of "multidose" packaging which has the enormous advantage of permitting the discontinuous and repeated use of the product without altering its characteristics and sterility.

On the contrary, because spray-form packaging is possible only for products having a particle size of not more than 20 microns, powdered collagen formulations are currently marketed only in the form of sachets and bottles. Such forms of packaging are of course not very suitable for the therapeutic uses of collagen: sachets, in addition to being difficult to meter, once opened cannot be re-used without impairing the sterility of the product.

According to the preferred embodiment of the invention, the diluted gel is atomised in an atomiser operating under the following conditions:
- temperature of the nozzle: 80-90°C
- pressure of the nebuliser: 1-3 bar
- temperature of the gel on entry: 80°C
- temperature of the air: 80°C
- temperature of the product on discharge: 65°C
- moisture content of the product on discharge: 10-15%.

The micronised collagen obtained under the conditions described above has the following characteristics:
- particle size: from 5 to 20 microns (98%),
- moisture content: not more than 16%.

The micronised collagen can then be packaged in the forms of administration known in the art, generally in combination with the normal excipients and coadjuvants; the preferred formulations are, for example, sachets of from 0.1 to 50 grams, bottles of from 0.5 to 250 grams, spray dispensers of from 10 to 1000 ml; in this last case it is of course necessary to add a suitable propellant gas, generally a preconstituted mixture of n-butane, isobutane and propane gases. The finished products are then subjected to a sterilisation treatment by the application of ionising radiation or other suitable sterilising systems.

### Example 1

500 litres of a 1.2% by w/v collagen solution are diluted with 1500 litres of distilled water (dilution ratio 1:4) in order to obtain 2000 litres at 0.4% by w/v. The pH of the solution is corrected to 4.5 ± 0.5 using dilute acetic acid.

The solution is then introduced into a test atomiser under the following operating conditions:
air and nozzle temperature: 80-85°C
temperature on entry: 80°C
temperature on discharge: 65°C
pressure of the nebuliser: 2 bar
capacity of the feed pump: 40 1/hour.

At the end of the operation, approximately 8.0 kilograms of powdered collagen having the desired particle size (98% < 20 microns) and moisture content (K.F. 14%) are collected.

### Example 2

The micronised collagen obtained in the manner described in Example 1 is packaged automatically in sachets of combined polyethylene/aluminium/paper material; using a dose of 0.1, 0.25, 0.5 and 1.0 gram per sachet.

The sachets so obtained are subjected to treatment with ionising radiation at a dose of 25 kilograys in order to obtain a powder completely free from micro-organisms.

### Example 3

The micronised collagen obtained in the manner described in Example 1 is packaged automatically in bottles of neutral glass having a cap and an under-cap of non-toxic plastics material; using doses of 1, 2 and 5 grams per bottle.

The bottles so obtained are subjected to treatment with ionising radiation at a dose of 25 kilograys in order to obtain a powder completely free from micro-organisms.

### Example 4

The micronised collagen obtained in the manner described in Example 1 is packaged automatically in aluminium spray dispensers having an internal lining of epoxy resin. 50 and 125 ml spray dispensers containing, respectively, 1 and 2 grams of micronised collagen are used. The spray dispensers are then equipped with delivery valves and the propellant composed of a preconstituted mixture of n-butane, isobutane and propane (95:2:3) is then introduced at a pressure of approximately 1.3 bar.

The spray dispensers so obtained are subjected to treatment with ionising radiation at a dose of 25 kilograys in order to obtain a powder completely free from micro-organisms.

## Claims

1. Use of micronised collagen having a particle size of not more than 20 microns and maintaining the quaternary aggregation form of native collagen, for the manufacture of a pharmaceutical composition for wound-healing.

2. Use according to claim 1, **characterised in that** said collagen is type I native collagen.

3. Use according to claim 1, **characterised in that** said collagen has a particle size of approximately 18 microns.

4. Use according to claim 1, **characterised in that** said composition is in the form of a spray.

## Patentansprüche

1. Verwendung eines mikronisierten Kollagens mit einer Partikelgröße von nicht mehr als 20 µm und die quaternäre Aggregierungsform von nativem Kollagen bewahrend, zur Herstellung einer pharmazeutischen Zusammensetzung zur Wundheilung.

2. Verwendung gemäß Anspruch 1, **dadurch gekennzeichnet, daß** das Kollagen natives Kollagen vom Typ I ist.

3. Verwendung gemäß Anspruch 1, **dadurch gekennzeichnet, daß** das Kollagen eine Partikelgröße von ungefähr 18 µm hat.

4. Verwendung gemäß Anspruch 1, **dadurch gekennzeichnet, daß** diese Zusammensetzung in Form eines Sprays vorliegt.

## Revendications

1. Utilisation de collagène micronisé ayant une taille de particules de pas plus de 20 µm et conservant la forme à structure quaternaire du collagène natif, pour la préparation d'une composition pharmaceutique pour la guérison des plaies.

2. Utilisation selon la revendication 1, **caractérisée en ce que** ledit collagène est du collagène natif de type 1.

3. Utilisation selon la revendication 1, **caractérisée en ce que** ledit collagène a une taille de particules d'environ 18 µm.

4. Utilisation selon la revendication 1, **caractérisée en ce que** ladite composition est sous la forme d'une pulvérisation.
